(19) Europäisches Patentamt
European Patent Office
Office européen des brevets

(11) Numéro de publication: **0 260 472 B1**

(12) **FASCICULE DE BREVET EUROPEEN**

(45) Date de publication de fascicule du brevet: **13.03.91**

(51) Int. Cl.5: **C07C 403/16**, C07C 45/45, C07C 49/557

(21) Numéro de dépôt: **87112138.0**

(22) Date de dépôt: **21.08.87**

(54) **Procédé pour la préparation de cétones cycloaliphatiques.**

(30) Priorité: **19.09.86 CH 3760/86**

(43) Date de publication de la demande: **23.03.88 Bulletin 88/12**

(45) Mention de la délivrance du brevet: **13.03.91 Bulletin 91/11**

(84) Etats contractants désignés: **CH DE FR GB LI NL**

(56) Documents cités:
**FR-A- 2 178 252**
**US-A- 4 226 892**

(73) Titulaire: **FIRMENICH SA**
**Case postale 239**
**CH-1211 Genève 8(CH)**

(72) Inventeur: **Fehr, Charles, Dr.**
**6, chemin Ravoux**
**CH-1290 Versoix(CH)**
Inventeur: **Galindo, José**
**c/o FIRMENICH SA Case Postale 239**
**CH-1211 Geneve 8(CH)**

(74) Mandataire: **Salvadori, Giuseppe, Dr.**
**c/o Firmenich S.A. Case Postale 239**
**CH-1211 Genève 8(CH)**

**EP 0 260 472 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La gamma-damascone, ainsi que la gamma-damascénone, ou 2-méthylène-6,6-diméthyl-1-crotonoyl-cyclohexane et 2-méthylène-6,6-diméthyl-1-crotonoyl-cyclohex-3-ène respectivement, au même titre que leurs isomères alpha et bêta et leurs dérivés à chaîne butanoùïque, constituent des ingrédients fort utiles pour l'industrie de la parfumerie et des arômes.

Il s'agit de composés de formule

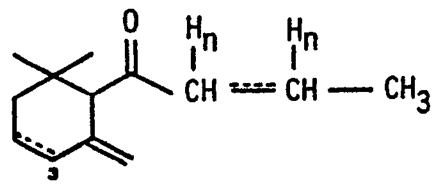

(I)

pouvant contenir une liaison supplémentaire dans les positions indiquées par les pointillés et dans laquelle l'indice n vaut zéro ou 1 [voir à cet effet par exemple : brevets USA n°s 3,928,456, 3,975,310 et 4,226,892].

La présente invention apporte une solution nouvelle au problème posé par leur synthèse.

Grâce au procédé de l'invention, il est désormais possible d'obtenir avantageusement les précurseurs de formule

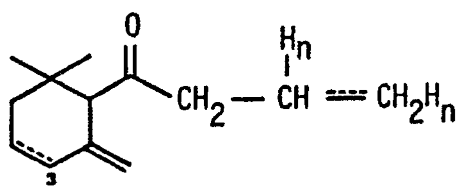

(IIb)

dans laquelle l'indice n et les pointillés ont le sens indiqué ci-dessus, lesquels composés peuvent être ensuite aisément transformés en les dérivés de formule (I) au moyen d'une isomérisation selon les méthodes connues, par exemple à l'aide d'un agent acide.

Un des objets de la présente invention consiste en un procédé pour la préparation des cétones cycloaliphatiques de formule

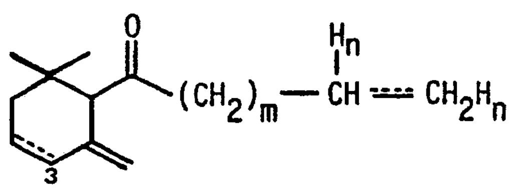

(II)

dans laquelle l'indice m sert à désigner un nombre entier égal à 0, 1 ou 2, n vaut zéro ou 1 et les pointillés indiquent une liaison supplémentaire éventuelle, lequel procédé est caractérisé en ce qu'on effectue successivement les étapes réactionnelles suivantes :

a. déprotonation d'un ester de formule

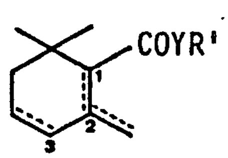

(III)

contenant soit une double liaison isolée dans la position 1 ou 2 (exocyclique), soit deux doubles liaisons conjuguées dans les positions 1 et 3 ou 2 (exocylique) et 3 telles qu'indiquées par les pointillés, et dans laquelle le symbole R' représente un reste alkyle, linéaire ou ramifié, de préférence $C_1$-$C_6$, ou phényle, substitué ou non, et Y sert à désigner un atome d'oxygène ou de soufre ;

b. addition à l'énolate ainsi formé de formule

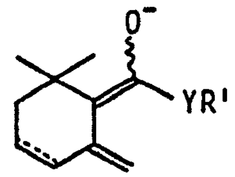

(IV)

d'un composé organométallique de formule

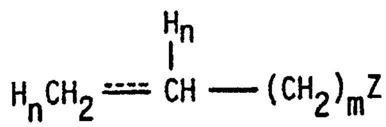

(V)

l'indice m désignant un nombre entier égal à 0, 1 ou 2, n définissant un nombre entier de valeur zéro ou 1, Z représentant un radical MgX ou un métal alcalin, de préférence le lithium, X désignant un atome d'halogène et la ligne pointillée une liaison supplémentaire facultative ;
c. hydrolyse du produit obtenu.

Schématiquement, le procédé défini ci-dessus peut être représenté ainsi :

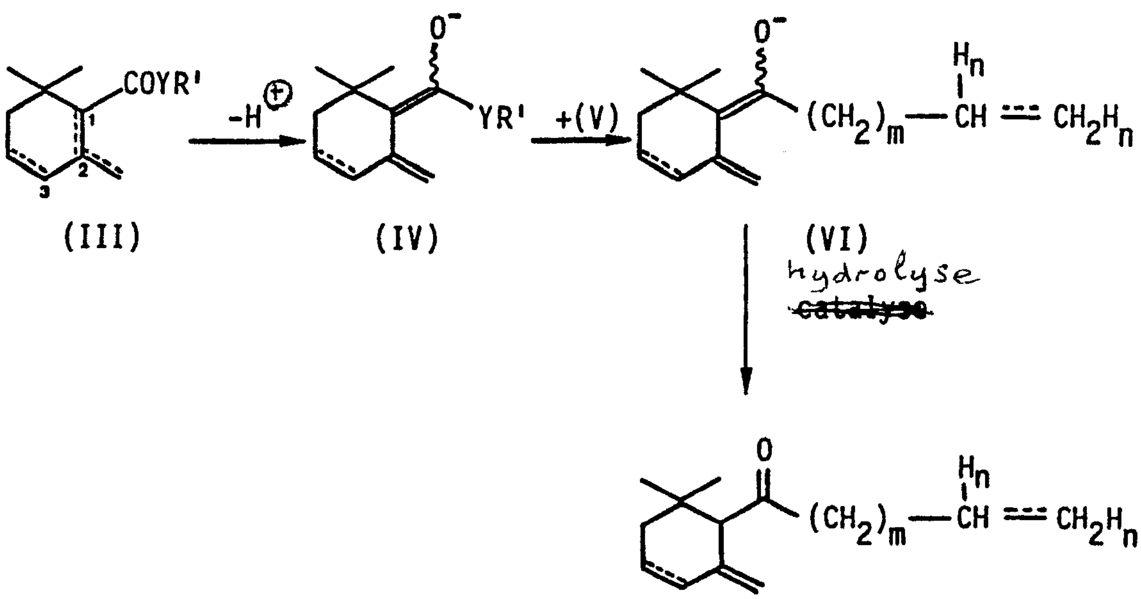


(III) (IV) (VI)

A titre de produit de départ de formule (III), on peut utiliser un ester cycloaliphatique choisi parmi les esters alkyles inférieurs de l'acide bêta ou gamma-cyclogéranique ou de l'acide bêta- ou gamma-safranique. A cet effet, il convient de mentionner le bêta- ou gamma-cyclogéraniate, ou le bêta- et gamma-safranate de méthyle, éthyle, n-propyle, iso-propyle ou butyle. Il s'agit de produits disponibles commercialement ou qui peuvent être facilement préparés par voie de synthèse conformément à des méthodes connues. A titre d'esters préférentiels, il convient de mentionner le bêta- ou gamma-cyclogéraniate de méthyle et le bêta-safranate de méthyle ou éthyle.

Comme produits de départ, on peut également employer des thioesters. A titre d'exemple, on peut citer parmi les thioesters le bêta-thiocyclogéraniate de S-phényle.

La première étape du procédé, qui consiste en la déprotonation desdits esters de départ, s'effectue à l'aide d'une base forte. Comme base forte, on peut utiliser un dérivé alkyle d'un métal alcalin, tel le propyl- ou le butyl-lithium, ou encore un amidure de métal alcalin, de préférence un amidure de lithium.

C'est ainsi que des bases telles le diméthyl-, le diéthyl- ou le diisopropyl-amidure de lithium constituent des bases préférentielles. Des mélanges constitués par deux des bases précitées peuvent également être employés.

La réaction s'effectue dans des solvants organiques inertes, par exemple en dissolvant l'ester de départ dans un éther, tel le tétrahydrofuranne et la base dans un hydrocarbure aliphatique, cycloaliphatique ou aromatique, tels par exemple l'hexane, le cyclohexane, le benzène ou le toluène.

Sans vouloir préjuger de l'exactitude de l'interprétation mécanistique de la réaction qui caractérise cette première étape de la réaction, on peut affirmer que le produit formé se présente sous forme d'une entité transitoire dans les conditions de la réaction. Plusieurs indices indirects tendent toutefois à en confirmer la structure. Il nous a été par exemple possible d'isoler le dérivé silylé de formule

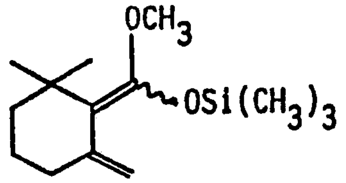

(IVb)

par adjonction du chlorure de triméthyl-silyle au mélange réactionnel, après traitement du gamma- ou bêta-cyclogéraniate de méthyle avec une base forte selon l'invention.

Ce fait suggère que l'entité transitoire se présente en bonne probabilité sous forme d'un énolate, tel qu'indiqué par la formule (IV), lequel pourrait trouver son origine soit dans la déprotonation directe de l'ester (III), soit dans l'action de la base forte utilisée (ou d'un nucléophile) sur un cétène-acétal de formule (IVb) suivant le schéma que voici :

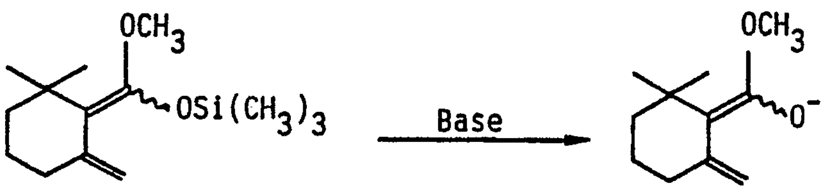


(IVb) (IV)

Quant à la température de réaction de cette première étape du procédé de l'invention, elle peut se situer aux environs de la température ambiante ou être légèrement inférieure à celle-ci, par exemple à environ 15-25° C. Selon un mode d'exécution particulier du procédé de l'invention, en utilisant du n-butyl-lithium comme base et du bêta-cyclogéraniate de méthyle comme ester de départ, on peut opérer à environ 15° C.

Des températures inférieures peuvent également être employées. Par exemple, le traitement du 2,6,6-triméthyl-1-cyclohex-1-ène-1-carbothioate de S-phényle par du butyl-lithium s'effectue aisément à la température de -78° C.

A titre de composé organométallique de formule (V), on peut employer des halomagnésiens d'alkyle ou d'allyle tel le chlorure ou bromure d'allyl-magnésium ou de méthyl-, éthyl-, propyl- ou butyl-lithium.

De tels composés sont obtenus au moyen des méthodes bien connues dans l'art à partir de l'halogénure d'alkyle ou d'allyle choisi, par réaction de ceux-ci avec du magnésium métallique, généralement en solution éthérée suivant les conditions de la réaction de Grignard. Comme réactif organométallique, on peut également employer des composés alkylés de métaux alcalins, par exemple le butyl-lithium, ce qui permet l'obtention de composés cétoniques (II) comportant une chaîne latérale saturée (n = 1) :

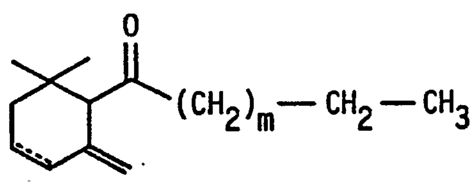

La dernière étape s'effectue par hydrolyse du produit obtenu par traitement de celui-ci avec de l'eau, de préférence en milieu légèrement acide, par exemple au moyen d'une solution aqueuse de chlorure d'ammonium glacée. Les traitements usuels de séparation des phases, neutralisation et distillation permettent l'obtention des produits désirés.

L'invention est illustrée de manière plus détaillée par les exemples suivants dans lesquels les températures sont indiquées en degrés centigrades et les abréviations ont le sens usuel.

Exemple 1

Préparation de 2-méthylène-6,6-diméthyl-1-but-3-en-1-oyl-cyclohexane

Dans un ballon à sulfuration de 4 l équipé d'une agitation mécanique et maintenu sous azote, on additionne pendant 10 min à une température d'environ 15-17° une solution de butyl-lithium dans l'hexane (520 ml d'une solution 1,45 N) à une solution de bêta-cyclogéraniate de méthyle (100 g ; 0,549 M) dans 1500 ml de tétrahydrofuranne (THF) anhydre. On laisse réagir 5 min à 15°, puis additionne pendant environ 3 min 320 ml d'une solution 2,40 N de chlorure d'allyl-magnésium dans THF (1,4 equivalents). La température monte progressivement et à 26° on refroidit avec un bain de glace de façon à maintenir la température de la réaction entre 30 et 35°. Après 10 min, on hydrolyse en versant le mélange sur une solution aqueuse de $NH_4Cl$/glace et, après séparation des phases, on lave la phase organique avec une solution aqueuse saturée de NaCl, puis le solvant est évaporé. On obtient par simple distillation deux fractions d'un produit brut, à savoir :

83,20 g à Eb. 65°/6,65 Pa (pureté 92%)

9,05 g à Eb. 65-75° /6,65 Pa (pureté 60%)

Rend. 80%.

Une purification ultérieure fournit un produit dont le point d'ébullition est de 87°/2,66 × $10^2$ Pa.

Le produit ainsi obtenu peut être transformé en gamma-damascone par isomérisation au moyen d'un traitement acide avec de l'acide ptoluènesulfonique.

En effectuant la réaction, dans des conditions identiques à celles qui ont été décrites dans l'exemple ci-dessus, sur du bêta-safranate de méthyle (20 g) à la place de bêta-cyclogéraniate de méthyle, on a obtenu 19,1 g de 2-méthylène-6,6-diméthyl-1-but-3-en-1-oyl-cyclohex-3-ène.

Le bêta-safranate de méthyle utilisé comme produit de départ dans le procédé décrit peut être préparé comme indiqué dans la demande de brevet européen n° 46 606.

Exemple 2

Préparation de 2-méthylène-6,6-diméthyl-1-pentan-1-oyl-cyclohexane

Une solution de 14,3 ml (0,020 M) de butyl-lithium dans l'hexane est ajoutée à -78° à une solution de 2,6 g de 2,6,6-triméthyl-1-cyclohexène-1-carbothioate de S-phényle (0,010 M). La température est ensuite laissée remonter jusqu'à température ambiante, puis, après 30 min environ, on effectue l'hydrolyse au moyen d'une solution aqueuse saturée de $NH_4Cl$, comme indiqué à l'exemple précédent. La phase organique est séparée, lavée jusqu'à neutralité et séchée. Après évaporation, on obtient par distillation 1,25 g de la cétone désirée ayant Eb. 100-150°/6,6 Pa. Le thioester de départ peut être préparé à partir du chlorure de 2,6,6-triméthyl-cyclohex-1-énoyl selon la méthode que voici.

Dans un ballon 3-cols maintenu sous azote, on additionne goutte à goutte 6,46 g (0,051 M) de chlorure d'oxalyle à une solution d'acide bêta-cyclogéranique (5,73 g ; 0,034 M) dans 60 ml de chlorure de méthylène. On chauffe ensuite à reflux jusqu'à cessation du dégagement gazeux. Les parties volatiles sont éliminées sous vide.

Dans un deuxième réacteur, on traite une solution de 3,57 g (0,032 M) de thiophénol dans 40 ml de tétrahydrofuranne avec une solution de butyl- lithium (22,5 ml ; 0,032 M). A la solution ainsi obtenue, on ajoute la solution du chlorure d'acide préparé séparément dans du THF tout en maintenant la température à 10°. Après une nuit à température ambiante, on verse le mélange réactionnel sur une solution à 5% de NaOH et glace. On extrait à l'éther, lave les extraits organiques puis, après les traitements usuels de séchage et évaporation, on distille pour obtenir 6,5 g du carbothioate de S-phényle désiré.

RMN ($CDCl_3$ ; 60 MHz) : 1,15 (6H, s) ; 1,55 (4H, m) ; 1,85 (3H, s) ; 1,7-2,1 (2H, m) ; 7,35 (5H, s) delta ppm.

En opérant de façon identique, on a préparé le 2,6,6-triméthyl-1-pentanoyl-cyclohex-2-ène à partir de 2,6,6-triméthyl-cyclohex-2-ène-1-carbothioate de S-phényle.

**Revendications**

**1.** Procédé pour la préparation des cétones cycloaliphatiques de formule

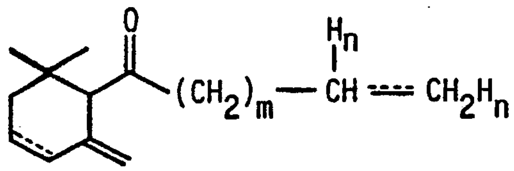

(II)

dans laquelle l'indice m sert à désigner un nombre entier égal à 0, 1 ou 2, n vaut zéro ou 1 et les pointillés indiquent une liaison supplémentaire éventuelle, caractérisé en ce qu'on effectue successivement les étapes réactionnelles suivantes :

a. déprotonation d'un ester de formule

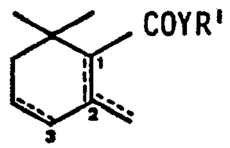

(III)

contenant soit une double liaison isolée dans la position 1 ou 2 (exocyclique), soit deux doubles liaisons conjuguées dans les positions 1 et 3 ou 2 (exocyclique) et 3 telles qu'indiquées par les pointillés, et dans laquelle le symbole R′ représente un reste alkyle linéaire ou ramifié, de préférence $C_1C_6$, ou phényle, substitué ou non, et Y sert à désigner un atome d'oxygène ou de soufre ;

b. addition à l'énolate ainsi formé de formule

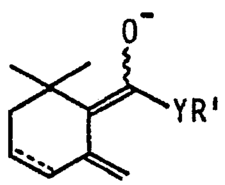

(IV)

d'un composé organométallique de formule

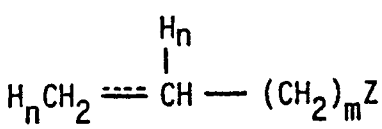

(V)

l'indice m désignant un nombre entier égal à 0, 1 ou 2, n définissant un nombre entier de valeur zéro ou 1, Z représentant un radical MgX ou un métal alcalin, de préférence le lithium, X désignant un atome d'halogène et la ligne pointillée une liaison supplémentaire facultative ;

c. hydrolyse du produit obtenu.

**2.** Procédé suivant la revendication 1, caractérisé en ce qu'on effectue la déprotonation par traitement de l'ester de formule (III) avec une base forte.

**3.** Procédé suivant la revendication 2, caractérisé en ce qu'on utilise comme base forte un dérivé alkyle d'un métal alcalin ou un amidure de métal alcalin.

**4.** Procédé suivant la revendication 3, caractérisé en ce qu'on utilise comme base forte le propyl- ou le butyl-lithium, ou le diméthyl-, diéthyl- ou le diisopropylamidure de lithium.

**5.** Procédé suivant l'une des revendications 2 à 4, caractérisé en ce qu'on utilise la base à raison d'une quantité supérieure à la quantité équivalente de l'ester de départ (III).

6. Procédé selon la revendication 1, caractérisé en ce qu'on utilise comme composé organométallique de formule (V) le chlorure d'allyl-magnésium.

7. Procédé selon la revendication 6, caractérisé en ce qu'on utilise le chlorure d'allyl-magnésium à raison d'une quantité supérieure à la quantité équivalente de l'ester de départ (III).

8. Procédé selon la revendication 1, caractérisé en ce qu'on utilise comme ester de départ de formule (III) le bêta- ou le gamma-cyclogéraniate de méthyle, comme base forte le butyl-lithium et comme composé organométallique le chlorure d'allyl-magnésium pour fournir, après hydrolyse, le 2méthylène-6,6-diméthyl-1-but-3-én-1-oyl-cyclohexane.

9. Procédé selon la revendication 1, caractérisé en ce qu'on utilise comme ester de départ de formule (III) le bêta-safranate de méthyle, comme base forte le butyl-lithium et comme composé organométallique le chlorure d'allyl-magnésium pour fournir, après hydrolyse, le 2-méthylène-6,6-diméthyl-1-but-3-én-1-oyl-cyclohex-3-ène.

## Claims

1. Process for the preparation of cycloaliphatic ketones of formula

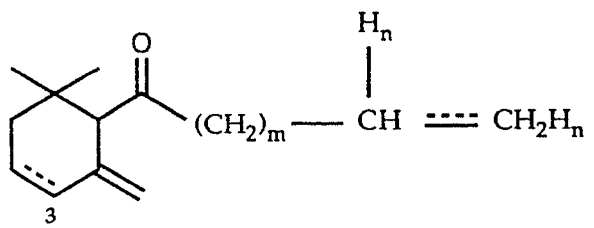

(II)

wherein index m designates integer 0, 1 or 2, n stands for 0 or 1 and the dotted lines represent an optional additional bond, characterized in that the following subsequent reaction steps are carried out:

a. deprotonation of an ester of formula

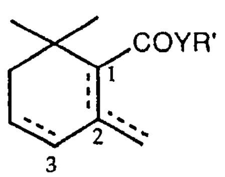

(III)

having either an isolated double bond in position 1 or 2(exocyclic), or two conjugated double bonds in positions 1 and 3 or 2(exocyclic) and 3 as indicated by the dotted lines, and wherein symbol R' represents a linear or branched alkyl radical, preferably a $C_1$-$C_6$ alkyl radical, or a substituted or unsubstituted phenyl, and Y designates an oxygen or a sulphur atom;

b. addition to the thus formed enolate of formula

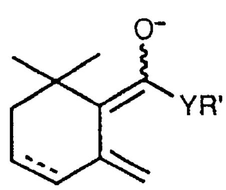

(IV)

of an organometallic compound of formula

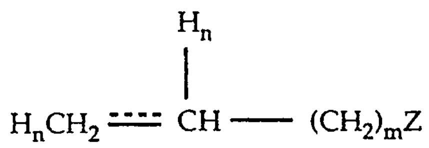

(V)

wherein m stands for integer 0, 1 or 2, n has the value of 0 or 1, Z represents a MgX radical or an alkali metal, preferably lithium, X designating a halogen atom, and the dotted line represents an optional additional bond;

c. hydrolysis of the resulting product.

2. Process according to claim 1, characterized in that the deprotonation is carried out by treating the ester of formula (III) with a strong base.

3. Process according to claim 2, characterized in that the strong base is an alkali metal alkyl derivative or an alkali metal amide.

4. Process according to claim 3, characterized in that the strong base is propyllithium, butyllithium, or lithium dimethyl-, lithium diethyl- or lithium diisopropyl-amide.

5. Process according to any of claims 2 to 4, characterized in that the base is used in an amount higher than the equivalent amount of starting ester (III).

6. Process according to claim 1, characterized in that the organometallic compound of formula (V) is allyl-magnesium chloride.

7. Process according to claim 6, characterized in that the allyl-magnesium chloride is used in an amount higher than the equivalent amount of starting ester (III).

8. Process according to claim 1, characterized in that the starting ester of formula (III) is methyl $\beta$- or $\gamma$-cyclogeranate, the strong base is butyllithium and the organometallic compound is allyl-magnesium chloride, and the obtained product after hydrolysis is 2-methylene-6,6-dimethyl-1-but-3-en-1-oyl-cyclohexane.

9. Process according to claim 1, characterized in that the starting ester of formula (III) is methyl $\beta$-safranate, the strong base is butyllithium and the organometallic compound is allyl-magnesium chloride, and the obtained product after hydrolysis is 2-methylene-6,6-dimethyl-1-but-3-en-1-oyl-cyclohex-3-ene.

## Ansprüche

1. Verfahren zur Herstellung von cycloaliphatischen Ketonen der Formel

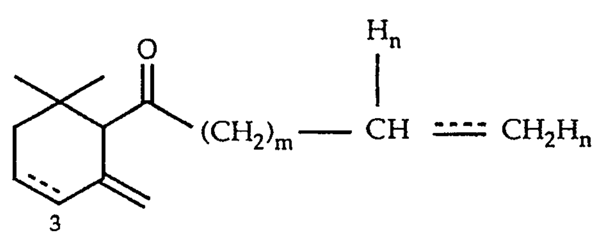

(II)

worin der Index m für die Zahl 0,1 oder 2 steht, n 0 oder 1 darstellt, und die gestrichelte Linie eine eventuelle zusätzliche Doppelbindung bedeutet, dadurch gekennzeichnet, daß man die folgenden Reaktionsstufen schrittweise durchführt:

a. Deprotonierung eines Esters der Formel,

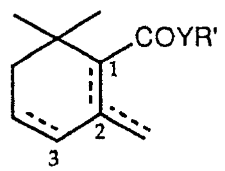

(III)

der entweder, wie durch die gestrichelten Linien angezeigt, eine alleinstehende Doppelbindung in der 1 oder 2 (exozyclischen) Stellung, oder zwei konjugierten Doppelbindungen in den 1 und 3 oder 2 (exozyclischen) und 3 Stellungen enthält, und worin das Symbol R' für einen linearen oder verzweigten, vorzugsweise $c_1$-$c_6$ Alkylrest, oder für einen gesättigten oder ungesättigten Phenylrest steht, und Y ein Sauerstoffatom oder ein Schwefelatom bedeutet;

b. Addierung einer organometallischen Verbindung der Formel,

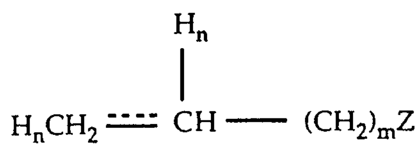

(V)

worin der Index m die Zahl 0,1 oder 2 bedeutet, n für 0 oder 1 steht, Z ein MgX Radical, in welchem X ein Halogenatom ist, oder ein Alkalimetall, vorzugsweise Lithium, darstellt, und die gestrichelte Linie eine wahlweise zusätzliche Bindung bezeichnet, an das so erhaltenene Enolat der Formel

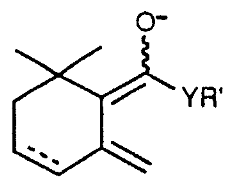

(IV)

c. Hydrolyse des erhaltenen Produktes.

**2.** Verfahren nach Anspruch 1, dadurch gekennzeichnet, daß man bei der Behandlung des Esters der Formel (III) die Deprotonierung mit einer starken Base bewirkt.

**3.** Verfahren nach Anspruch 2, dadurch gekennzeichnet, daß man als starke Base, ein Alkylderivat eines Alkalimetalls, oder ein Alkalimetall Amid verwendet.

**4.** Verfahren nach Anspruch 3, dadurch gekennzeichnet, daß man als starke Base, Propyl- oder Butyllithium, oder Lithium Dimethyl-, Diäthyl-oder Diisopropyl-amid verwendet.

**5.** Verfahren nach einem der Anprüche 2 bis 4, dadurch gekennzeichnet, daß die Base in einer grösseren Menge als das Äquivalent des Ausgangsesters (III) verwendet wird.

**6.** Verfahren nach Anspruch 1, dadurch gekennzeichnet, daß man als organometallische Verbindung der Formel (V), Allylmagnesiumchlorid verwendet.

**7.** Verfahren nach Anspruch 6, dadurch gekennzeichnet, daß das Allylmagnesiumchlorid in einer grösseren Menge als das Äquivalent des Ausgangsesters (III) verwendet wird.

**8.** Verfahren nach Anspruch 1, dadurch gekennzeichnet, daß man als Ausgangsester der Formel (III), β- oder γ-Methylcyclogeraniat, als starke Base, Butyllithium und, als organometallische Verbindung, Allylmagnesiumchlorid verwendet, um nach Hydrolyse 2-Methylen-6,6-dimethyl-1-(but-3-en-1-oyl)-cyclohexane zu erhalten.

**9.** Verfahren nach Anspruch 1, dadurch gekennzeichnet, daß man als Ausgangsester der Formel (III), β-Methylsafranat, als starke Base, Butyllithium, und, als organometallischen Verbindung, Allylmagnesiumchlorid verwendet, um nach Hydrolyse 2-Methylen-6,6-dimethyl-1-(but-3-en-1-oyl)-cyclohex-3-en zu erhalten.